# EUROPÄISCHE PATENTANMELDUNG

(11) **EP 0 737 479 A1**
(43) Veröffentlichungstag der Anmeldung: **16.10.1996**
(21) Anmeldenummer: 95890086.2
(22) Anmeldetag: 13.04.1995
(51) Int. Cl.: A61K 35/78, A61K 7/04, A61K 7/043, A61K 7/48, A01N 65/00

(54) **Mittel zur Pflege des Felles, der Haut und gegebenenfalls der Hufe von Tieren, insbesondere von Pferden**

(71) Anmelder: Haerpfer-Horn, Herta, Mag., A-1040 Wien (AT); Haerpfer, Christian, Dr., A-1040 Wien (AT)
(72) Erfinder: Haerpfer-Horn, Herta, Mag., A-1040 Wien (AT); Haerpfer, Christian, Dr., A-1040 Wien (AT)
(74) Vertreter: Itze, Peter, Dipl.-Ing.

(57) **Zusammenfassung**

Die Erfindung bezieht sich auf ein Mittel zur Pflege des Felles, der Haut und gegebenenfalls der Hufe von Pferden, sowie zum Fernhalten von insbes. fliegenden Insekten, wobei als Wirksubstanzen ätherische Öle eingesetzt sind, wobei ätherische Öle wenigstens einer Pflanze der Gattung der Myrthengewächse und/oder der Lippenblütler in einer aus pflanzlichen Ölen gebildeten Trägerflüssigkeit gelöst sind, und wobei das Verhältnis von ätherischen Ölen zu Trägerflüssigkeit insbesondere von etwa 1 : 18 bis 1 : 4,5, vorzugsweise 1 : 9, beträgt.

## Beschreibung

Die Erfindung bezieht sich auf ein Mittel zur Pflege des Felles, der Haut und gegebenenfalls des Hufes von Pferden, sowie zum Fernhalten von insbes. fliegenden Insekten, wobei als Wirksubstanzen ätherische Öle eingesetzt sind.

Insbesondere in der warmen Jahreszeit, in welcher das Pferd bei seiner Arbeit leicht zu schwitzen beginnt, kommt es durch das oftmalige Waschen des Pferdes, insbesondere mit Reinigungsmitteln, Haarwaschmitteln u.dgl. zu einer starken Verminderung des normalen Fettgehaltes des Pferdefelles. Dadurch kann es zu einem Verlust des Glanzes des Felles, aber auch zu einem Auftreten von Juckreiz usw. kommen, wobei auch eine vermehrte Schuppenbildung und sogar Hautkrankheiten auftreten können. Auch durch in der warmen Jahreszeit vielfach applizierte Mittel zum Vertreiben von Insekten, insbesondere fliegenden Insekten, wie Stallfliegen, Bremsen und anderen stechenden Insekten, kann es zu einer Verminderung des Fettgehaltes des Felles kommen, da diese Mittel vielfach als Trägersubstanzen alkoholische Flüssigkeiten besitzen. Diese alkoholischen Flüssigkeiten lösen dann das Fett aus dem Fell bzw. der darunterliegenden Haut heraus, wodurch dann wieder die bereits angesprochenen Fellschädigungen auftreten können.

Es war daher Ziel der Erfindung, nunmehr ein Mittel der eingangs genannten Art zu schaffen, welches einerseits dem Vermindern des Fettgehaltes vorbeugt und anderseits auch gleichzeitig eine insektenvertreibende Wirkung aufweist, wobei die Wirkung von ätherischen Ölen sich für diese Verwendung angeboten hat.

Es hat sich nun gezeigt, daß derartige ätherische Öle auch bei der Pflege von Tieren, u.zw. sowohl bei der Fell- als auch bei der Haut- und Hufpflege, eine entsprechende Wirkung erbringen können, u.zw. dann, wenn in erfindungsgemäßer Weise ätherische Öle wenigstens einer Pflanze der Gattung der Myrthengewächse und/oder der Lippenblütler in einer aus rein pflanzlichen Ölen gebildeten Trägerflüssigkeit gelöst sind, wobei das Verhältnis von ätherischen Ölen zur Trägerflüssigkeit insbesondere von etwa 1 : 18 bis etwa 1 : 4,5, vorzugsweise 1 : 9, beträgt. Die aus pflanzlichen Ölen gebildete Trägerflüssigkeit wirkt dabei einerseits als Lösungsmittel und anderseits auch als Transfermittel für die ätherischen Öle in die Haut, da die Trägerflüssigkeit von der Haut aufgenommen wird, u.zw. ohne die Poren oder die Hautoberfläche zu verstopfen. Die ätherischen Öle der Gattung der Myrthengewächse und/oder Lippenblütler wirken dabei sowohl insektenvertreibend als auch allgemein hautpflegend bzw. wirken sie auch gegen Parasitenbefall, wie Pilze oder Milben, und dienen auch zur rascheren Heilung kleinerer Wunden. Gerade diese Wirkstoffkombination ist für Pferde wichtig, weil dadurch erreicht wird, daß bei kleineren Verletzungen, die sich Pferde beim Spiel, auf der Koppel oder im Stall zufügen können, die Infektionsgefahr verringert wird. Aus den angeführten Mengenverhältnissen ist ersichtlich, daß die ätherischen Öle in der Regel nur etwa 10 % der gesamten Flüssigkeit betragen, wobei die pflanzlichen Öle neben der Lösungs- und Transportfunktion der ätherischen Öle auch noch die bekannte pflegende Funktion auf Haut und Haar des Felles der Pferde aufweisen, wobei sich auch herausgestellt hat, daß eine pflegende Wirkung auch im Hufbereich, insbesondere im Saumband und am Kronenrand des Hufes, erzielt wird.

Das erfindungsgemäße Mittel hat gegenüber den herkömmlichen insektenvertreibenden oder insektenvernichtenden Mitteln noch den Vorteil, daß es aufgrund der Aufnahme der ätherischen Öle in die Haut eine wesentlich längere Wirkungsdauer als die herkömmlichen Mittel aufweist, wobei die Mittel, die auf chemisch-synthetischer Basis arbeiten, zudem auch noch sehr hautreizend sind. Die vielfach angewendeten natürlichen Mittel, nämlich verdünnte Essigsäurelösungen, haben den Nachteil, daß sie nur eine sehr begrenzte Wirksamkeitdauer haben, da diese Mittel sehr leicht flüchtig sind. Außerdem ist bei diesen bekannten Mitteln auch noch der Nachteil gegeben, daß bei einem Schwitzen des Pferdes der Schweißgeruch den abwehrenden Geruch des Mittels übertüncht, sodaß durch den Schweißgeruch die Insekten erneut angelockt werden.

Überdies haben Mittel mit einem aufdringlichen Geruch noch den Nachteil, daß sie unter gewissen Umständen die Pferde irritieren, sodaß es beim Ausreiten zu ungewollten Reaktionen des Pferdes, wie Scheuen od.dgl., kommen kann.

All diese Nachteile werden durch die angeführten erfindungsgemäßen Mitteln vermieden, da eben aufgrund der Aufnahme des Mittels in die Haut dieses sich nur langsam verflüchtigt, wodurch das Auftreten von übertrieben starken Geruchsbeeinträchtigungen nicht auftritt.

Die Wirkung des Mittels kann hinsichtlich seiner Eigenschaften noch dadurch verbessert werden, daß der Anteil an ätherischen Ölen auch solche von Pflanzen der Gattung der Gräser und/oder der Rautengewächse und/oder der Storchenschnabelgewächse und/oder der Holzgewächse enthält. Es können damit die speziellen pflegenden Eigenschaften bzw. auch die insektenvertreibenden Wirkungen genau gesteuert werden. Als Pflanzen der Gattung der Myrthengewächse werden dabei Nelke, Cajeput, Eukalyptus, als Pflanzen der Gattung der Lippenblütler Pfefferminze, Basilikum, Lavendel und/oder Rosmarin verwendet. Weiters können als Pflanzen der Gattung der Gräer Lemongras, der Rautengewächse Zitrone, der Hölzer Zeder und/oder Zypresse und der Storchenschnabelgewächse Geranien verwendet werden. Eine besonders gute und schonende Pflegewirkung kann dadurch erreicht werden, daß als pflanzliche Öle kaltgepreßte Pflanzenöle, z.B. Olivenöle od.dgl. verwendet sind.

Bei einem einfachen Mittel der erfindungsgemäßen Zusammensetzung kann sich der Anteil aus ätherischen Ölen wie folgt zusammensetzen: Eukalyptus 45 %, Pfefferminze 45 %, Lavendel 10 %. Dabei kommt der Eukalyptuspflanze und der Pfefferminze im wesentlichen die Wirkung der Vertreibung der Insekten zu, wobei Lavendel zur Desinfektion bzw. zur Behandlung der Insektenstiche dient. Eukalyptus begünstigt aber auch die Wundheilung, wobei die pflegende Wirkung zusätzlich dann von den aus pflanzlichen Ölen gebildeten Trägerflüssigkeiten vorgenommen wird.

Bei einem weiteren erfindungsgemäßen Mittel kann sich der Anteil an ätherischen Ölen wie folgt zusammensetzen: Eukalyptus 24 %, Pfefferminze 24 %, Lavendel 6 %, Nelke 29 %, Rosmarin 5 %, Zitrone 12 %. Zu den bereits angeführten Wirkungen von Eukalyptus, Pfefferminze und Lavendel kommt noch die keimtötende und entzündungshemmende Wirkung der Nelke hinzu, welche auch den Juckreiz nach Parasitenstichen lindert. Auch Rosmarin hilft der Wundheilung, ist entzündungshemmend und wirkt gegen Parasiten, wobei Rosmarin auch eine allgemeine hautpflegende Wirkung aufweist. Zitrone wiederum hat neben der Hautpflege auch eine keratinkräftigende Wirkung und auch Pflege, was insbesondere dem Fell zugute kommt. Überdies wirkt die Zitrone juckreizstillend und keimtötend, was sich im Falle von Parasitenbefall natürlich sehr positiv auswirkt.

Eine bevorzugte Zusammensetzung des erfindungsgemäßen Mittels ist folgende: Basilikum 8 %, Cajeput 6 %, Eukalyptus 12 %, Geranie 7 %, Lavendel 4 %, Lemongras 12 %, Nelke 20 %, Pfefferminze 12 %, Rosmarin 3 %, Zeder 4 %, Zitrone 8 %, Zypresse 4 %. Zusätzlich zu den bereits vorstehend angeführten Wirkungen kommt noch die Wirkung der Geranie hinzu, welche ebenfalls auf ihre Weise hautpflegend ist und gegen Parasiten wirkt. Weiters ist der Wirkstoff der Geranie schmerzstillend bzw. wundheilend und entzündungshemmend. In analoger Weise wirkt auch das Lemongras, die Zeder, die Zypresse, Basilium und Cajeput.

Zusammenfassend kann bei diesem Mittel angeführt werden, daß dieses Mittel in der Gesamtheit auch einen beruhigenden Einfluß auch auf sehr nervöse Pferde aufweist.

Schließlich kann das erfindungsgemäße Mittel zusätzlich noch bis zu 10 % Natriumchlorid enthalten, was dazu dient, den Schweißgeruch etwas zu neutralisieren, wodurch vermieden wird, daß aufgrund des Schweißgeruches die Insekten angezogen werden.

Wie aus Vorstehendem hervorgeht, wird das erfindungsgemäße Mittel unter Verzicht auf Hinzufügung synthetischer Substanzen hergestellt, sodaß seine Wirkung ausschließlich auf den Eigenschaften und den Inhaltsstoffen der verwendeten Öle liegt, u.zw. sowohl der ätherischen Öle als auch der Trägeröle. Diese ätherischen Öle sind somit absolut unschädlich und völlig ungiftig und haben damit keinerlei Nebenwirkungen. Trotzdem bietet es einen völligen Schutz vor Belästigung und Stichen von Fliegen, Bremsen, Gelsen, Mücken u.dgl., wobei zusätzlich gleichzeitig das Fell des Pferdes entsprechend gepflegt wird, da mit diesem Mittel die erforderliche Rückfettung der Haut erfolgt, wenn das Pferd wie im Sommer üblich, des öfteren mit schärferen Shampoos gewaschen wird. Der Schutz liegt dabei in der Verhinderung jedweden Hautkontaktes der Insekten mit dem gesamten Tierkörper und hält einige Stunden an, u.zw. sowohl im freien Gelände als auch im Stallbereich.

Das erfindungsgemäße Mittel wird mittels eines Tuches aufgetragen, was das Pferd nicht irritiert, wie es im Falle des Auftragens mittels Spraydosen od.dgl. der Fall ist. Außerdem ist, wie schon angeführt, der Geruch des erfindungsgemäßen Mittels eher beruhigend und wird sowohl vom Menschen als auch vom Pferd als nicht unangenehm empfunden. Dies zeigt sich auch darin, daß selbst bei nervösen Pferden der Kopf ohne Schwierigkeiten mit dem erfindungsgemäßen Mittel bestrichen werden kann, ohne daß das Pferd scheut.

Die Wirkung des erfindungsgemäßen Mittels kann dabei in folgenden Punkten zusammengefaßt werden:
Schutz des Pferdes vor der penetranten Belästigung durch die Fliegen während seiner Ruhestunden in den Stallungen,
Schutz des Pferdes vor der enormen Fliegenbelästigung und vor Bremsenstichen bei Koppelbenutzung und damit ungestörte Erholung,
Schutz des Pferdes vor durch den ständigen Hautkontakt mit Fliegen hervorgerufenen Krankheiten, wie z.B. Augenentzündungen,
Schutz eines operierten oder verletzten Pferdes durch Ermöglichung einer störungsfreien Genesungsphase,
Schutz des Pferdes vor Stechinsekten, insbes. Bremsen, bei Ausritten im Gelände,
Schutz des Reiters vor den Folgen einer Panikreaktion des Pferdes nach einem schmerzhaften Stich einer Pferdebremse,
Schutz des Pferdes durch regelmäßige Pflege von Fell und Haut,
Schutz des Pferdes durch Auftragen der Mixtur am Huf, was sich äußerst pflegend auf Kronrand, Saumband und Ballen auswirkt.

Auf die chemische Zusammensetzung und die Wirkung der einzelnen ätherischen Öle braucht in diesem Zusammenhang nicht näher eingegangen zu werden. Wichtig ist, daß das erfindungsgemäße Mittel neben der starken insektenabweisenden Wirkung noch einem Parasitenbefall, wie Milben, Pilze u.dgl., vorbeugt bzw. die befallenen Hautbereiche von diesen Infektionen befreit.

Das erfindungsgemäße Mittel kann im übrigen auch auf Wunden, die in der Heilungsphase sind, aufgebracht werden, da dieses Mittel keinerlei irritierende Wirkung auf sensible Hautbereiche aufweist, sondern vielmehr aufgrund adstringierender Wirkungen einzelner Bestandteile einen schnelleren Heilungsprozeß bewirkt. Außerdem wird verhindert, daß sich auf die Wunden Fliegen und andere Insekten setzen können und gegebenenfalls sogar Eier darin ablegen können, was zu weiteren Infektionen führen könnte. Außerdem sind - wie schon angeführt - die Öle teilweise starke Antiseptika und haben daher antibiotische, entzündungshemmende und antivirale Eigenschaften, was entzündungshemmend, vernarbungsprozeßfördernd und auf die Haut tiefenreinigend wirkt.

Sollte aufgrund eines zu starken Auftrages des Öles ein Fetten des Felles auftreten, dann kann das erfindungsgemäße Mittel durch einfaches Waschen jederzeit wieder entfernt werden.

## Patentansprüche

1. Mittel zur Pflege des Felles, der Haut und gegebenenfalls der Hufe von Pferden, sowie zum Fernhalten von insbes. fliegenden Insekten, wobei als Wirksubstanzen ätherische Öle eingesetzt sind, dadurch gekennzeichnet, daß ätherische Öle wenigstens einer Pflanze der Gattung der Myrthengewächse und/oder der Lippenblütler in einer aus pflanzlichen Ölen gebildeten Trägerflüssigkeit gelöst sind, wobei das Verhältnis von ätherischen Ölen zu Trägerflüssigkeit insbesondere von etwa 1 : 18 bis 1 : 4,5, vorzugsweise 1 : 9, beträgt.

2. Mittel nach Anspruch 1, dadurch gekennzeichnet, daß der Anteil an ätherischen Ölen auch solche von Pflanzen der Gattung der Gräser und/oder der Rautengewächse und/oder der Storchenschnabelgewächse und/oder der Holzgewächse enthält.

3. Mittel nach Anspruch 1 oder 2, dadurch gekennzeichnet, daß als Pflanzen der Gattung der Myrthengewäschse die Nelke, Cajeput, Eukalyptus und als Pflanzen der Gattung der Lippenblütler Pfefferminze, Basilikum, Lavendel, Rosmarin verwendet sind.

4. Mittel nach Anspruch 2, dadurch gekennzeichnet, daß als Pflanze der Gattung der Gräser Lemongras, der Rautengewächse Zitrone, der Hölzer Zeder und/oder Zypresse, und der Storchenschnabelgewäschse Geranie verwendet sind.

5. Mittel nach einem der Ansprüche 1 bis 4, dadurch gekennzeichnet, daß als pflanzliche Öle kaltgepreßte Pflanzenöle, z.B. Olivenöl od.dgl. verwendet sind

6. Mittel nach einem der Ansprüche 1 bis 5, dadurch gekennzeichnet, daß sich der Anteil an ätherischen Ölen zusammensetzt aus Eukalyptus 45 %, Pfefferminze 45 % und Lavendel 10 %.

7. Mittel nach einem der Ansprüche 1 bis 4, dadurch gekennzeichnet, daß sich der Anteil an ätherischen Ölen zusammensetzt aus: Eukalyptus 24 %, Pfefferminze 24 %, Lavendel 6 %, Nelke 29 %, Rosmarin 5 % und Zitrone 12%.

8. Mittel nach einem der Ansprüche 1 bis 4, dadurch gekennzeichnet, daß sich der Anteil an ätherischen Ölen zusammensetzt aus: Basilikum 8 %, Cajeput 6 %, Eukalyptus 12 %, Geranie 7 %, Lavendel 4 %, Lemongras 12 %, Nelke 20 %, Pfefferminze 12 %, Rosmarin 3 %, Zeder 4 %, Zitrone 8 % und Zypresse 4 %.

9. Mittel nach einem der Ansprüche 1 bis 8, dadurch gekennzeichnet, daß es zusätzlich bis zu 10 % NaCl enthält.
